# FASCICULE DE BREVET EUROPEEN

(11) **EP 1 003 837 B1**
(45) Date de publication et mention de la délivrance du brevet: **11.06.2003**
(21) Numéro de dépôt: 98933707.6
(22) Date de dépôt: 24.06.1998
(51) Int. Cl.: C12N 1/04, C12N 11/02

(54) **COMPOSITION POUR LA CONSERVATION DE QUANTITES DETERMINEES ET REPRODUCTIBLES DE MICRO-ORGANISMES, ET PROCEDE D'OBTENTION**
ZUSAMMENSETZUNG ZUR QUANTITATIVEN UND REPRODUZIERBAREN KONSERVIERUNG VON MICROORGANISMEN UND HERSTELLUNSVERFAHREN DAFÜR
COMPOSITION FOR PRESERVING PREDETERMINED AND REPRODUCIBLE AMOUNTS OF MICRO-ORGANISMS, AND METHOD FOR OBTAINING SAME

(30) Priorité: 25.06.1997 FR 9707954
(43) Date de publication de la demande: 31.05.2000
(73) Titulaire: INSTITUT PASTEUR DE LILLE, 59019 Lille Cédex (FR)
(72) Inventeur: HERNANDEZ, Jean-François, F-59650 Villeneuve d'Ascq (FR); PALUSZKIEWICZ, Fabrice, F-59000 Lille (FR); OUDART, Eric, F-59130 Lambersart (FR)
(74) Mandataire: Martin, Jean-Jacques
(86) Numéro de dépôt international: FR9801335
(87) Numéro de publication internationale: WO99000485

(56) Documents cités:
- EP-A- 0 346 545
- EP-A- 0 515 237
- EP-A- 0 569 623
- US-A- 3 567 585
- US-A- 5 545 555

## Description

La présente demande est relative à une composition pour la conservation de quantités déterminées et reproductibles de micro-organismes.

Elle est en outre relative à des pastilles lenticulées comprenant une telle composition.

Elle a également pour objet un procédé de fabrication de ces pastilles.

La présence de benzène dans l'eau minérale Perrier, de Listeria dans les fromages ou l'épizootie d'encéphalopathie spongiforme bovine ont eu un retentissement considérable. Les entreprises dans les domaines de l'eau, de l'agro-alimentaire ou de la santé, positionnées sur des secteurs sensibles, ont des clients très attentifs à la qualité des produits qui leur sont fournis. Aussi, la prévention des situations de crise est-elle vitale. En effet, un incident, tel qu'une contamination, peut avoir des répercussions graves sur la santé des consommateurs et des conséquences économiques désastreuses pour les entreprises (production gâchée, détérioration de l'image). De ce fait, le risque d'une contamination constitue une préoccupation majeure pour les pouvoirs publics qui instaurent des contraintes réglementaires, nationales, européennes ou internationales, de plus en plus fortes en termes de sécurité microbiologique et les industriels qui mettent en place des plans d'assurance qualité incluant en particulier la recherche des points critiques de contamination dans les procédés.

Aussi, de très nombreux laboratoires publics ou privés réalisent, chaque année, plusieurs milliers d'analyses chimiques et microbiologiques dans les domaines de l'agro-alimentaire, de l'environnement ou de la santé.

Des études interlaboratoires ont clairement démontré que les laboratoires travaillaient généralement avec une assez bonne précision mais que des écarts importants existaient entre les résultats obtenus par les différents laboratoires.

Ainsi, des résultats par excès peuvent être observés dans des laboratoires présentant des étuves mal réglées alors que des résultats par défaut sont observés dans des laboratoires utilisant des milieux de culture de mauvaise qualité.

Ces résultats erronés peuvent avoir un impact économique ou sur la santé publique important. Les résultats par excès peuvent conduire à refuser à tort un lot de produits, programmer inutilement, des travaux d'assainissement pour améliorer la qualité d'une plage, ou inquiéter sans raison la population en interdisant la consommation de l'eau du robinet.

Inversement, des résultats par défaut peuvent faire encourir un risque aux consommateurs en autorisant, la vente de produits contaminés, la distribution d'eau mal désinfectée ou la baignade dans une eau polluée.

Afin de garantir la fiabilité des mesures, il a été demandé aux laboratoires de contrôle d'être accrédités. Initialement, cette accréditation définissait uniquement des obligations de moyens (personnel qualifié, locaux adaptés, maintenance des appareils de mesure, contrôle des matières premières et écriture des procédures analytiques). Actuellement, elle exige en plus des obligations de résultats. Cette nouvelle exigence impose la mise en place d'un système de qualité comprenant des contrôles internes et la participation à des essais inter-laboratoires.

Pour la mise sous contrôle des chaînes analytiques en interne et l'organisation d'essais inter-laboratoires, il est impératif de disposer de matériaux de référence. Ces matériaux de référence doivent être stables dans le temps et doivent permettre de reconstituer aisément des échantillons de contrôle homogènes et renfermant une quantité précise du produit à doser.

En chimie, ces matériaux de référence existent depuis de nombreuses années. Par contre, dans le domaine de la microbiologie, il est particulièrement difficile de conserver des quantités précises de micro-organismes. En effet, le nombre de micro-organismes peut augmenter, si le milieu de conservation permet leur multiplication au cours de la fabrication , du transport ou du stockage, ou diminuer, s'ils meurent durant la fabrication, le transport, le stockage ou au moment de la reconstitution de l'échantillon contrôle avant l'emploi.

Des méthodes de conservation de micro-organismes comme la lyophilisation et le cryoconservation ont déjà été décrites dans l'état de la technique. Néanmoins, ces méthodes entraînent, au moment du conditionnement, des taux de mortalité importants parmi les micro-organismes conservés, et en conséquence ne permettent pas de maîtriser précisément la quantité finale de micro-organismes survivants.

Ainsi, deux brevets faisant appel à une méthode de congélation brutale en azote liquide ne décrivent pas la conservation de bactéries, ou de micro-organismes et n'abordent pas le problème des dosages quantitatifs.

Le brevet US- 5 364 756 décrit une méthode de cryoconservation de cellules eucaryotes. Une suspension de ces cellules est préparée dans un tampon comprenant des agents cryoprotecteurs, puis la solution est nébulisée par ultrasons afin de former des microgouttelettes qui sont rapidement refroidies et séchées.

Le brevet US-5 405 616 concerne la conservation de molécules pharmacologiquement actives. Les particules comprenant ces molécules sont constituées principalement d'une macromolécule hydrophile soluble dans l'eau formant un squelette. Diverses protéines sont mentionnées, principalement des protéines d'origine végétale.

Des quantités précises de micro-organismes peuvent être obtenues à partir d'échantillons naturellement ou artificiellement contaminés. Cependant, la conservation de tels échantillons ne peut dépasser 24 heures et exige une température inférieure à 10°C.

Des quantités précises en diverses bactéries ont également été obtenues après congélation dans un milieu contenant du sérum et de l'inositol (1993 Peterz et Steneryd, J. of Appl. Bacteriol., 74, 143-148) ou dans du lait écrémé ( 1994, Schijven, Havellaar et Bahar, Appl. and Environ. Microbiol., 60, 4160-4162). Il s'avère cependant que ces suspensions bactériennes congelées ne sont pas stables au-delà de trois mois pour le premier type de préparation et d'un an pour le second. De plus, elles exigent des conditions de transport très contraignantes (délais brefs et maintien de la température en dessous de -70°C). Enfin leur stabilité en cas de décongélation et de recongélation successives n'a pas été démontrée.

Actuellement, seule l'atomisation réussit à concilier les contraintes de conservation, de stabilité et de transport avec l'exigence de disposer d'une quantité précise de micro-organismes.

Des suspensions de *Bacillus cereus* dans du lait concentré, atomisées et encapsulées dans de la gélatine, ont été utilisées comme matériaux de référence après transport à température ambiante (Paul H. Int Veld, 1993, Int. J. of Food Microbiol.,20, 23-36).

Cependant, cette technique est d'une mise en oeuvre difficile car elle nécessite une dissolution de la gélatine dans un milieu de reconstitution maintenu à 37°C. De plus certaines espèces comme *Aeromonas hydrophila, Pseudomonas aeruginosa* et *Campylobacter jejuni* ne peuvent être conservées de manière stable par atomisation. Cette méthode n'est pas non plus recommandée pour la conservation de pathogènes du système respiratoire transmissibles par aérosols comme *Legionella pneumophila.* Enfin, les matériaux de référence préparés par atomisation ont un coût élevé.

Il ressort donc de l'état de la technique que l'on ne connaissait pas de méthode facile à mettre en oeuvre et peu coûteuse permettant de conserver de manière stable des quantités précises de micro-organismes.

Le demandeur a résolu ce problème en trouvant une composition particulière permettant de conserver la viabilité des micro-organismes.

La présente invention est donc relative à une composition pour la conservation en quantités déterminées et reproductibles de micro-organismes comprenant en combinaison des quantités efficaces d'au moins une substance susceptible de former le squelette de pastilles lenticulées et d'au moins une substance saturante ainsi que des micro-organismes.

Pour la présente invention, on entend par quantités reproductibles de micro-organismes des variations du ratio entre deux mesures comprises, dans 95% des cas, entre 0,25 et 4 et encore plus préférentiellement entre 0,5 et 2.

Ladite composition comprend entre environ 10 et 60%, de substances susceptibles de former le squelette des pastilles lenticulées. Préférentiellement on utilise un mélange d'albumine et d'amidon, contenant entre environ 20 et 40% d'albumine et entre environ 2 et 5% d'amidon (en poids total de la composition). Après congélation et séchage ces molécules forment un squelette fortement poreux et en même temps mécaniquement stable, qui se dissout rapidement dans l'eau.

De manière avantageuse, l'albumine rentrant dans cette composition est de l'ovalbumine. L'ovalbumine peut être en particulier sous la forme d'une préparation de blanc d'oeuf. Le blanc d'oeuf constitue un milieu stérile et riche en protéines protectrices au sein duquel il est possible de disperser de manière homogène les micro-organismes. Elle peut néanmoins être toute autre albumine (sérum albumine bovine), ou toute protéine, présentant la même fonction.

L'amidon peut être tout type d'amidon, et en particulier des amidons naturels ainsi que modifiés, des dextranes, des dextrines et de la maltodextrine. Il peut être substitué par toute autre macromolécule hydrophile, en particulier des protéines végétales, ou leurs hydrolysats, des collagènes, des gélatines, de l'hydrolysat d'élastine ou des mélanges des substances précitées.

Ladite composition comprend également entre environ 40 et 90% de substance saturante. La matière saturante peut être constituée d'un ou plusieurs sels ou sucres mélangés. Ces sels ou sucres peuvent être ajoutés sous forme de poudre, de saumure ou de sirop. En utilisant du saccharose, préférentiellement entre environ 60 et 80%, la composition présente une activité en eau inférieure à 0,9. De ce fait, les dommages cellulaires occasionnés par la congélation et le séchage sont limités, et le développement des micro-organismes est empêché en particulier lors d'un transport éventuel à température ambiante.

Selon une mise en oeuvre de l'invention, la composition contient un ou plusieurs types de micro-organismes. Ils peuvent être des bactéries anaérobies strictes, aéro-anaérobies ou aérobies strictes, des bactéries psychrophiles, mésophiles ou thermophiles, des bactéries halophiles, des entérobactéries, des streptocoques, des staphylocoques, des bactéries pathogènes (*Salmonella, Campylobacter, Yersinia, Listeria, Pseudomonas aeruginosa, Aeromonas, Vibrio,* etc.), des levures ou des moisissures ou champignons, des bactériophages ou des virus, ou des kystes de protozoaires.

De manière avantageuse la présente composition contient entre 10² et 10¹¹, et préférentiellement entre 10² et 10⁹ micro-organismes par gramme. Une telle composition peut être avantageusement mise sous forme de pastilles, et en particulier de pastilles de forme lenticulée, présentant un diamètre compris entre 1 et 10 mm.

De telles pastilles présentent avantageusement une masse comprise entre environ 1 et 250 mg, préférentiellement entre environ 2 et 100 mg et encore plus préférentiellement entre 10 et 25 mg.

Les pastilles selon la présente invention peuvent être avantageusement obtenues par un procédé de fabrication comprenant les étapes suivantes:
a) incorporation des micro-organismes à la substance squelette;
b) diminution de l'activité en eau par ajout progressif de la substance saturante;
c) mise en forme des pastilles, et
d) séchage des pastilles sous vide et à une température inférieure à environ -10°C.

De manière préférentielle, le mélange est effectué de la manière suivante:
a) mélange de l'albumine et des micro-organismes;
b) ajout de l'amidon, puis
c) ajout du saccharose.

Plusieurs modes de séchage sont envisageables. Avantageusement, le séchage des pastilles est effectué dans un dessiccateur durant entre environ 12 heures et 10 jours.

En vue d'une stabilité optimale, c'est-à-dire sur une durée de plus de 12 mois, les pastilles peuvent être stockées à -70°C en présence d'un sachet déshydratant. Elles peuvent néanmoins être conservées 3 mois à -20°C et 4 jours à température ambiante, ce qui facilite notamment leur transport puisqu'aucune condition particulière et compliquée de transport n'est requise.

Un milieu de reconstitution approprié pour la mise en oeuvre de l'invention doit limiter au maximum le choc osmotique au moment de la dissolution des pastilles. Une solution de sel de mer synthétique à 23 g/l est recommandée comme reconstituant, car elle présente un niveau de récupération des micro-organismes plus élevé que les diluants habituellement employés en microbiologie que sont le Ringer, la peptone saline ou l'eau distillée. Une telle solution peut être celle commercialisée par la Société Aquarium Systems (MENTOR, OHIO, USA) sous la dénomination Instant Ocean, ou le milieu DSM commercialisé par la Société SANOFI PASTEUR (Marnes la Coquette, FRANCE).

La dissolution des pastilles doit s'effectuer à température ambiante. Si l'échantillon n'est pas analysé dans la demie heure qui suit la reconstitution, il est recommandé de le maintenir dans la glace fondante, afin de garantir une bonne stabilité.

Ces pastilles contenant des micro-organismes stables dans le temps, homogènes et susceptibles d'être reconstituées sous forme de suspension, de manière reproductible, c'est-à-dire avec des quantités variant faiblement d'un essai à l'autre constituent de véritables matériaux de référence particulièrement adaptés au contrôle de la fiabilité des mesures (contrôle interne et externe de qualité) dans le domaine de la bactériologie des eaux, des boissons et des produits alimentaires en général de la pharmacie, de la cosmétique, et de l'environnement.

Ce type de matériau de référence peut également être utilisé de façon avantageuse pour les « challenge tests » dans le domaine de l'agro-alimentaire. Pour cette utilisation particulière, les pastilles contiennent un ou plusieurs micro-organismes. Les pastilles peuvent être directement introduites dans le produit à tester (barquette, sachet de salade, plat cuisiné, etc.). Les produits sont ensuite placés dans les conditions réelles de conservation et l'évolution de la contamination introduite de manière quantifiée dans les produits est suivie selon les méthodes de dénombrement habituelles.

Plus généralement, ce nouveau type de matériau de référence peut être utilisé pour l'analyse microbiologique quantitative:
- contrôle de présence/absence de micro-organismes
- contrôle de la fertilité des milieux de culture
- contrôle d'efficacité d'antibiotiques, de substances bactéricides ou bactériostatiques,
- contrôle de stérilisation (ultra-violets, chloration, ozonisation, filtration, etc.).

Selon un autre aspect de l'invention, les pastilles comprenant au moins un type de micro-organismes, peuvent être avantageusement utilisées dans certains produits en tant qu'agents de démarrage pour l'ensemencement des souches en vue de maîtriser un paramètre microbiologique crucial intervenant dans un procédé biotechnologique, et en particulier de fermentation.

Ainsi, des pastilles contenant certaines levures, telles que par exemple *S. cerevisae, S. calbergensis, S. ellipsoidus*, peuvent être utilisées pour la fabrication du pain, de la bière haute et basse pression, du vin et autres alcools.

Les pastilles contenant des bactéries lactiques peuvent avantageusement être utilisées pour la fabrication de laits fermentés et de yaourt, ainsi que dans l'industrie pharmaceutique pour la fabrication de produits destinés au réensemencement de la flore intestinale.

Des pastilles contenant des spores fongiques activées, peuvent être utilisées directement ou après dilution à une teneur désirée en micro-organismes, dans l'industrie alimentaire, en particulier en fromagerie ou en salaison. Lesdites compositions peuvent par exemple être répandues ou incorporées dans les produits à transformer tels les fromages ou les produits de salaison.

Ainsi, des pastilles contenant certains genres bactériens, tels que par exemple *Rhizobium, Pseudomonas, Bacillus, Arthrobacter, Serratia* et *Azospirillum,* peuvent être utilisées en agriculture et dans l'industrie de l'environnement.

La présente invention est illustrée sans pour autant être limitée par les exemples qui suivent.

Les figures suivantes illustrent en outre la présente invention .

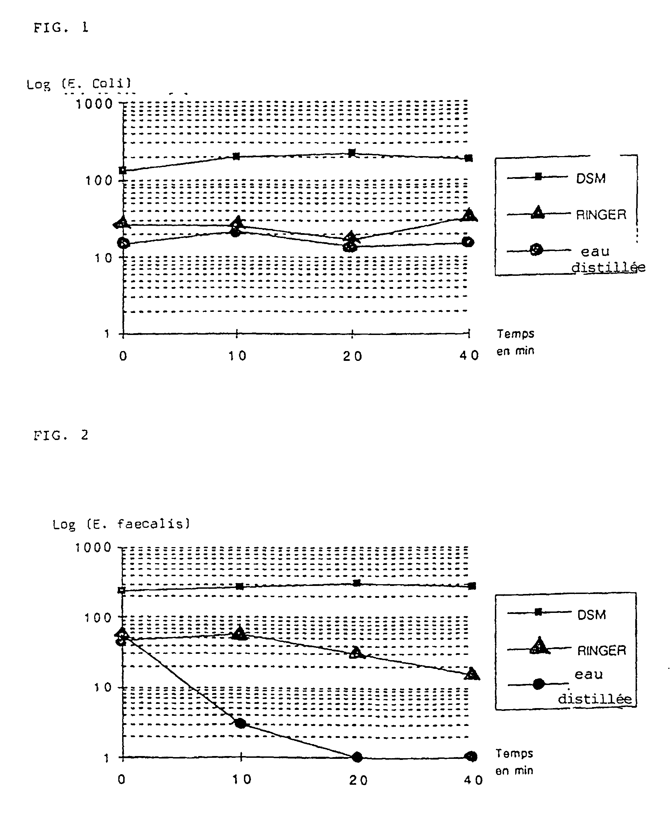

Les figures 1 et 2 illustrent la reconstitution de pastilles lenticulées contenant respectivement des bactéries *Escherichia coli* et *Enterococcus faecalis,* dans de l'eau distillée, du milieu de RINGER et du milieu DSM.

En ordonnées sont portées les valeurs Log (*E. coli*) pour la figure 1 et Log (*E. faecalis*) pour la figure 2.

La figure 3 est une analyse statistique des résultats de reconstitution obtenus avec *Escherichia coli* pour chacun des trois milieux de reconstitution. Cette figure illustre l'effet du milieu de reconstitution avec étude du rendement.

Les figures 4A à 4C illustrent la stabilité de pastilles lenticulées contenant *Escherichia coli* et stockées à respectivement +4°C, à -20°C et à -70°C. Ainsi, les figures 4A et 4C, illustrent l'effet de la température de stockage avec étude de la stabilité des matériaux de référence, avec pour la figure 4A *E. coli* conservé à + 4°C, pour la figure 4B *E. coli* conservé à -20°C et pour la figure 4C *E.coli* conservée à -7O°C.

Les figures 5A et 5B illustrent la stabilité de pastilles lenticulées contenant *Klebsiella planticola* et stockées à respectivement + 4°C et à - 20°C. Ainsi, les figures 5A et 5B illustrent l'effet de la température de stockage avec étude de la stabilité des matériaux de référence avec pour la figure 5A *Klebsiella planticola* conservé à +4°C et pour la figure 5B *Klebseilla planticola* conservée à -20°C.

Les figures 6A à 6C illustrent la stabilité de pastilles lenticulées contenant *Staphylococcus aureus* stockées respectivement à +4°C, -20°C et -70°C.Ainsi, les figures 6A à 6C illustrent l'effet de la température de stockage avec étude de la stabilité des matériaux de référence, cette figure correspondant à *Staphylococcus aureus* conservé à + 4°C, -20°C et -70°C.

Les figures 7A à 7C illustrent la stabilité de pastilles lenticulées contenant *Enterococcus faecalis* stockées respectivement à +4°C, -20°C et - 70°C.

Les figures 8A et 8B représentent les cartes de contrôle respectivement de la moyenne et de l'écart type obtenues pour le dénombrement d'*Escherichia coli* sur une période de plus de 30 jours.

Les figures 9A et 9B représentent les cartes de contrôle respectivement de la moyenne et de l'écart type obtenues pour le dénombrement d'*Enterococcus faecalis* sur une période de plus de 30 jours.

Les figures 10A et 10B représentent les cartes de contrôle respectivement de la moyenne et de l'écart type obtenues pour *S. aureus* sur une période de 30 semaines .

Les figures 11A et 11B représentent les cartes de contrôle respectivement de la moyenne et de l'écart type obtenues pour *Clostridium sporogenes* sur une période de 30 semaines.

### EXEMPLE 1:

### FABRICATION DE PASTILLES LENTICULEES.

### 1. Fabrication des pastilles

Des bactéries, sous forme de bouillons de culture, ou de colonies remises en suspension dans 0,25 ml de milieu DSM sont mélangées durant 3 min. dans 10 ml de blanc d'oeuf (environ 15g).

1.2g d'amidon sont alors ajoutés et l'ensemble est mélangé durant 30 secondes.

32g de sucre glace sont progressivement ajoutés tout en maintenant le brassage durant 7 min.

Enfin, le brassage est prolongé durant 3 mn sans ajout.

Le brassage est effectué pour ces différentes étapes dans un mixeur réglé à 700 t/min.

La composition ainsi obtenue est alors répartie en gouttes sur une plaque plastique, à l'aide d'une seringue de 10 ml munie d'une aiguille de 1,2 x 40mm.

La plaque est placée dans un dessiccateur, puis l'ensemble est mis sous vide et placé à -20°C durant 4 jours.

Les pastilles lenticulées, ou lenticules, ainsi obtenues sont récupérées puis stockées à + 4°C ou -20°C dans un flacon muni d'un sachet dessiccateur.

### 2. Echantillon bactérien

L'échantillon bactérien peut être ajouté au mélange sous deux formes:
- sous forme de bouillon;
- sous forme de colonies récupérées sur boîte.

L'échantillon sous forme de bouillon est utilisé lorsque la mortalité après séchage a été jugée importante lors d'un premier essai de conditionnement sous forme de lenticules. En effet le bouillon permet après centrifugation une concentration importante de bactéries difficilement réalisable avec des colonies.

L'échantillon sous forme de colonies a l'avantage d'être très facilement quantifiable. Il est utilisé lorsque le taux de survie après séchage est jugé correct. Son utilisation implique l'ajout de 0,25 ml de DSM, ce qui évite la modification des quantités vis-à-vis du mélange utilisant le bouillon.

### 3. Séchage du lot de pastilles.

Un essai de séchage à différentes températures d'un même lot de lenticule d'*E.coli* a été effectué.

### Mode opératoire:

- 10 gouttes d'un même lot de fabrication sont placées pour séchage à +44°C
- 10 autres gouttes sont placées pour séchage sous hotte à flux laminaire à température ambiante.
- les 10 dernières gouttes sont placées à -20°C à l'intérieur d'un dessiccateur dans lequel le vide a été réalisé.

Après 2 jours de séchage, les lenticules sont reconstituées dans un tube de 9 ml de milieu de RINGER.

5 ml de chacun des tubes sont ensuite filtrés sur milieu Tergitol TTC (AFNOR T 90-414).

### Résultats

Les résultats sont exprimés dans le tableau 1 ci-après.

Ces résultats montrent que le séchage à -20°C à l'intérieur d'un dessiccateur sous vide permet d'obtenir une viabilité supérieure aux autres méthodes de séchage.

### EXEMPLE 2

### RECONSTITUTION DE SOLUTIONS BACTERIENNES A PARTIR DES PASTILLES LENTICULEES.

### 1. Stabilité de l'échantillon reconstitué à température ambiante.

### a) E.coli ( souche WR₁ disponible auprès de la Collection Hollandaise RIVM).

### Mode opératoire:

- 4 lenticules d'*E. coli* obtenues selon l'exemple 1 sont placées respectivement dans des tubes contenant les milieux reconstituants DSM, RINGER et Eau Distillée Stérile;
- 2 ml de chaque tube sont ensuite versés à différents intervalles de temps (0, 10, 20, 40 minutes) dans un flacon (type Pasteur microbio) contenant 400 ml d'eau du robinet.

Les résultats sont résumés dans la figure 1.
- 2 répliquats de filtration sont effectués sur chaque flacon (filtration automatique de 100 ml sur automate SARAH).

On remarque pour *E. coli*:
- Une stabilité dans chacun des reconstituants;
- Un nombre de colonies beaucoup plus important (environ 10 fois plus) pour la lenticule reconstituée dans le DSM par rapport aux deux autres lenticules reconstituées respectivement dans le RINGER et dans l'Eau Distillée Stérile.

### b) Enterococcus faecalis.

(souche disponible auprès de la Collection Tchèque CCM sous le n° 2541).

### Mode opératoire:

- une lenticule d'*Enterococcus faecalis* obtenue selon l'exemple 1 est placée dans un tube de chaque reconstituant (DSM, RINGER, Eau Distillée Stérile);
- 1 ml de chaque tube est ensuite versé à différents intervalles de temps (0, 10, 20, 40 minutes) dans un flacon (type Pasteur microbio) contenant 400 ml d'eau du robinet;
- 2 répliquats de filtration sont effectués sur chaque flacon (filtration automatique de 100 ml sur automate SARAH).

La figure 2 illustre les résultats obtenus.

On remarque donc pour *Enterococcus faecalis:*
- dans de l'eau distillée stérile, une chute rapide du nombre de colonies récupérées en fonction du temps de contact de la lenticule avec le reconstituant (passage de 55 à 0 colonies récupérées en 20 minutes).
- dans le RINGER, une chute lente du nombre de colonies récupérées en fonction du temps de contact de la lenticule avec le reconstituant (passage de 47 à 15 colonies récupérées en 40 minutes).
- dans le DSM, une stabilité du nombre de colonies récupérées en fonction du temps avec un niveau de récupération beaucoup plus élevé.

### 2. Rendement des différents reconstituants

Ces essais ont pour but de montrer que le milieu DSM permet une meilleure récupération de colonies par rapport à d'autres diluants comme le RINGER ou l'eau distillée stérile.

### Mode opératoire:

- 10 comprimés d*'E.coli* obtenus selon l'exemple 1 sont reconstitués dans chacun des trois reconstituants (DSM, RINGER, eau distillée stérile).
- L'ensemble des tubes est placé à +4°C (eau + Ice Pack®) pour éviter tout effet de température,
- Le contenu de chaque tube est intégralement déversé dans un flacon de 400 ml d'eau du robinet (flacon de type Pasteur microbio),
- Tous les flacons sont ensuite filtrés par filtration automatique de 100 ml sur l'automate SARAH.

### Résultats:

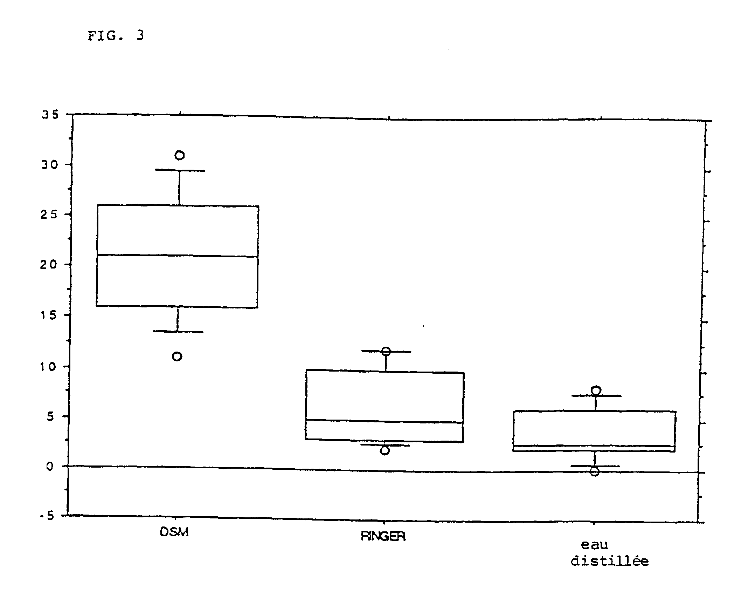

Ces résultats sont résumés dans la figure 3.

On note que :
- Pour le DSM, on récupère en moyenne environ 22 colonies par filtration;
- Pour le RINGER, on récupère en moyenne environ 7 colonies par filtration;
- Pour l'eau distillée stérile, on récupère en moyenne environ 3 colonies par filtration.
   Ainsi, pour le même lot de lenticules d'*E. Coli* , on récupère environ 3 fois plus de colonies lorsque le DSM est utilisé comme reconstituant par rapport au RINGER, et environ 7 fois plus de colonies par rapport à l'eau distillée stérile.
   Ces résultats (2.1 et 2.2) montrent donc que le DSM offre une meilleure stabilité et un meilleur rendement lors de la reconstitution des lenticules.
   Chacune des manipulations effectuées à l'aide de lenticules doit être préférentiellement effectuée à froid (pour une stabilité garantie au-delà de 40 minutes) et en milieu DSM.

### EXEMPLE 3:

### VALIDATION DES LOTS DE LENTICULES

Cinq souches d'*Escherichia coli*, de *Klebsiella planticola* (souche disponible auprès de l'ATCC sous le n° 33.531), d'*Enterococcus faecalis,* de *Straphylococcus aureus,* et *Clostridium sporogenes* ont été conditionnées sous forme de lenticules, tel que décrit dans l'exemple 1.

### Mode opératoire:

La validation s'effectue en dénombrant 30 lenticules après séchage.

Chacune des lenticules est déposée dans un tube contenant 9 ml de DSM.

Les tubes précédemment à température ambiante sont placés à +4°C (Bain d'eau + Ice Pack®) dès que la lenticule s'est dissoute dans le DSM (dissolution au Vortex®).

Une dissolution est effectuée lorsque la concentration de la lenticule est trop importante.

On filtre ensuite 5 ml de chacune des dilutions.

### Résultats:

Les résultats sont contenus dans le tableau 2 ci-après. Ces résultats montrent que pour tous les germes étudiés, le coefficient de reproductibilité (SR) est inférieur à 0,20 en unités logarythmiques. SR mesure la variabilité des résultats obtenus quand les mesures sont effectuées sur des pastilles différentes au sein d'un même laboratoire.

### EXEMPLE 4:

### STABILITE DES LOTS STOCKES.

### Mode opératoire:

Les lenticules sont conservées à + 4°C, -20°C ou -70°C dans des flacons munis d'un sachet dessiccateur (un même lot est séparé en deux quantités identiques, stockées à deux températures différentes).

Chaque jour deux lenticules sont testées , cela pour chaque lot et pour chacune des températures de conservation (+4°C, - 20°C et -70°C).

Chaque lenticule est placée dans un tube contenant 10 ml de DSM, celle-ci est dissoute au Vortex® à température ambiante. Les échantillons ainsi reconstitués sont placés à + 4°C avant la filtration.

La filtration s'effectue en deux répliquats de 5 ml pour chaque lenticule.

On a ainsi chaque jour la somme de quatre dénombrements pour chacune des deux températures de conservation ( + 4°C, - 20°C et - 70°C).

C'est le suivi de cette valeur qui permet d'établir la droite de régression permettant la visualisation de l'évolution dans le temps du lot.

### Résultats:

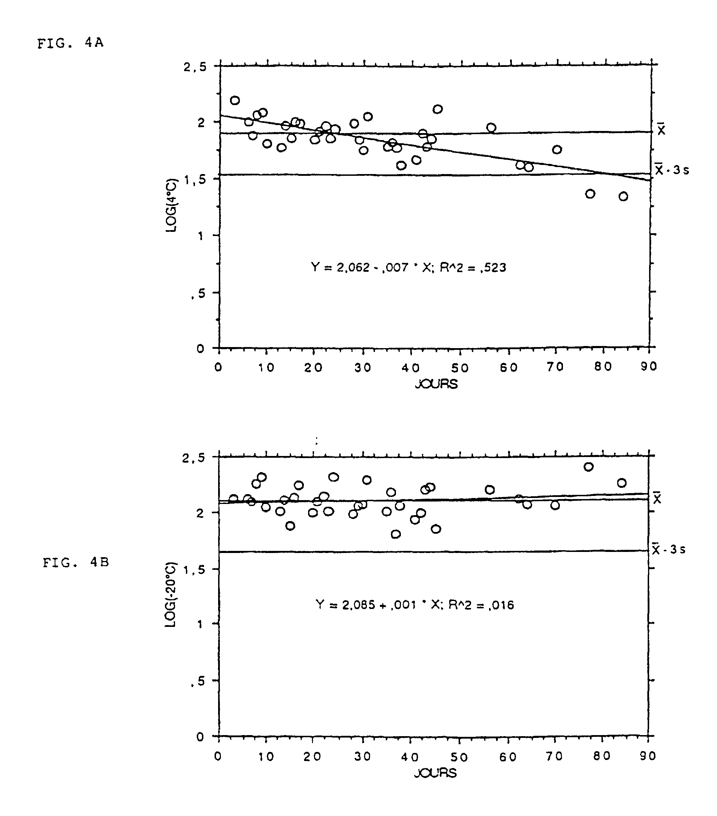

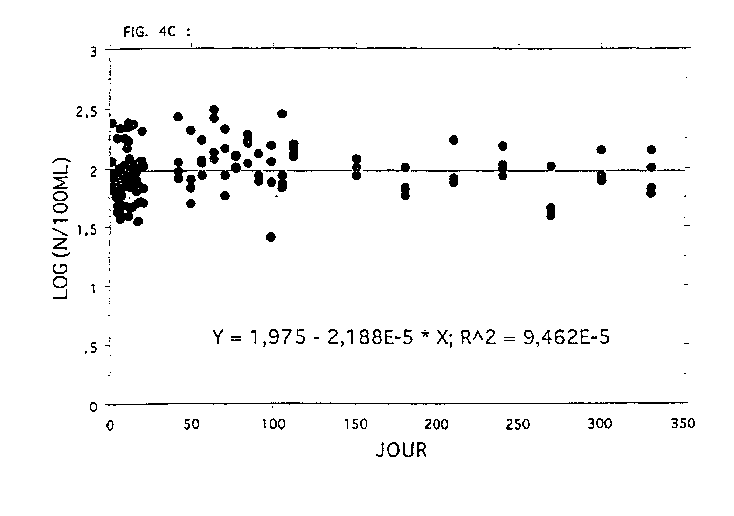

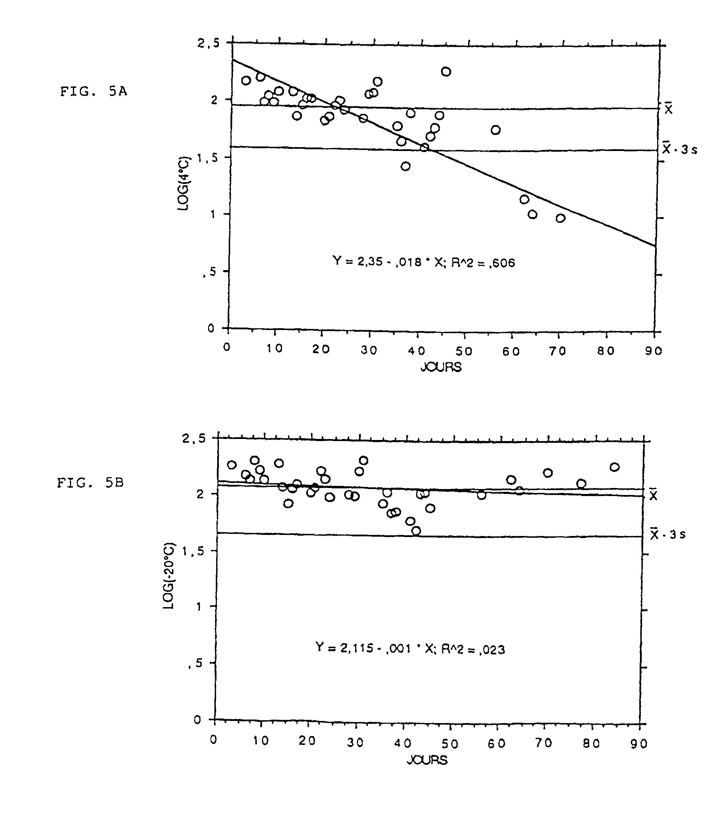

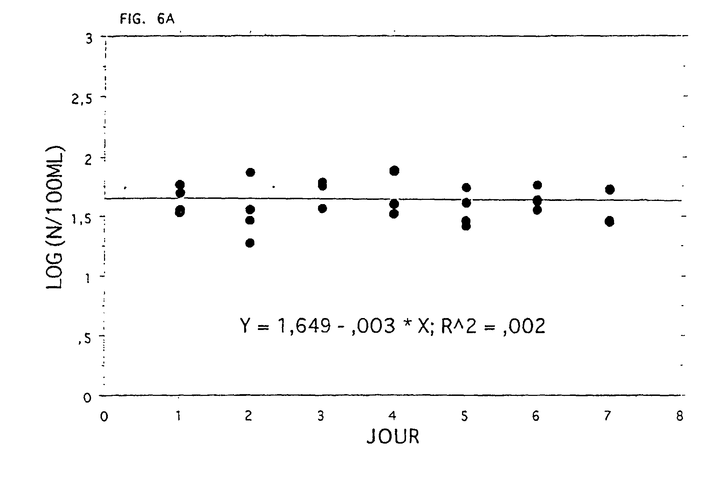

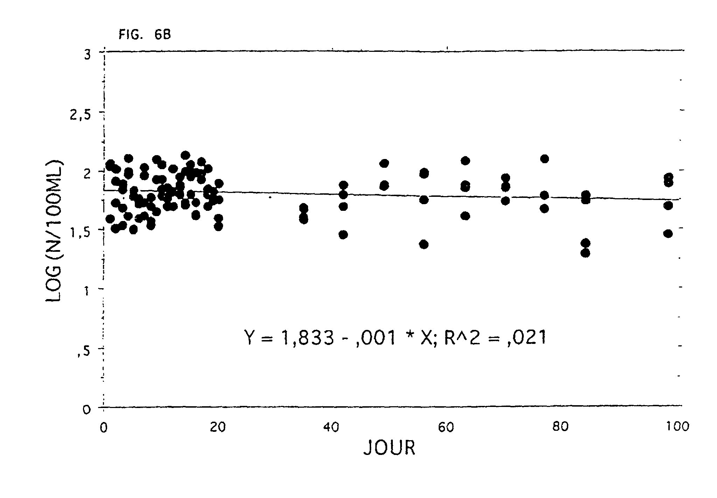

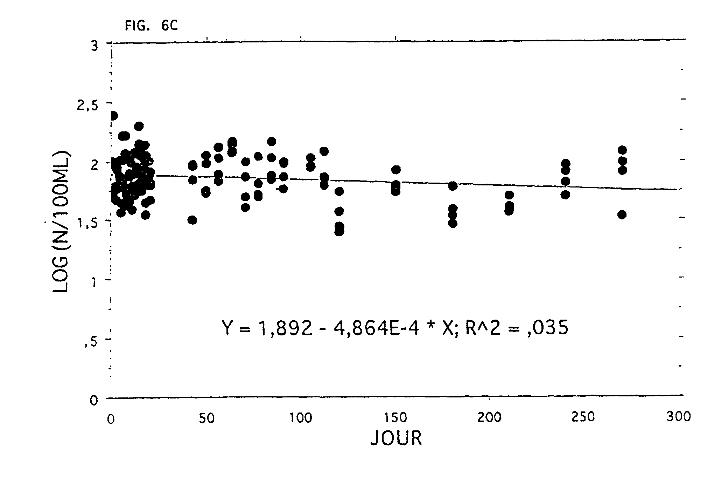

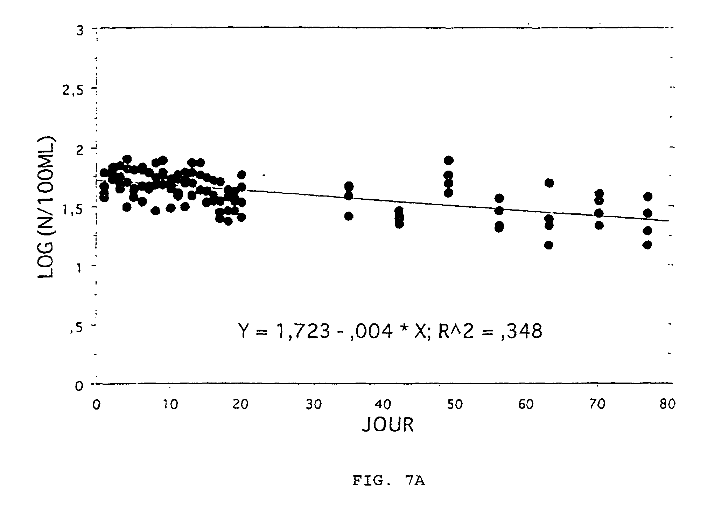

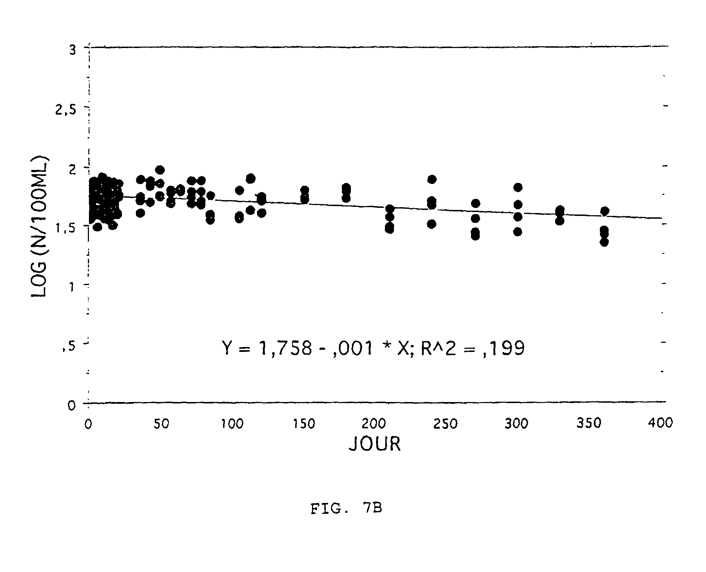

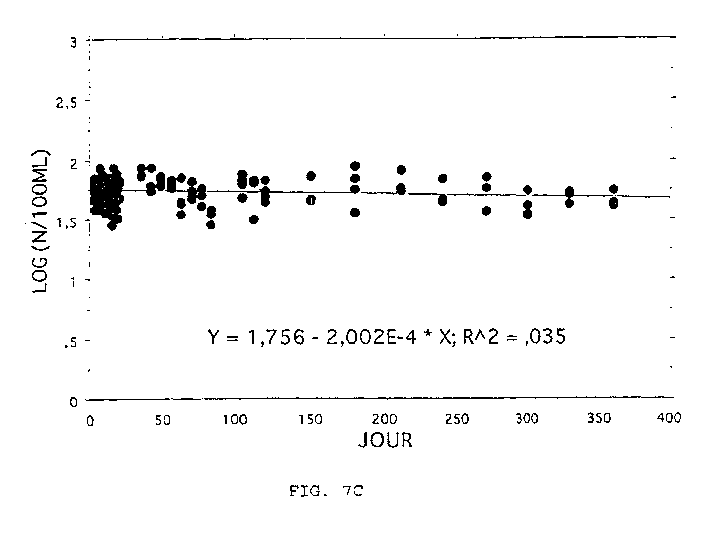

Les résultats de stabilité sont représentés sous forme de droites de régression pour :
- *E. coli* (Figs. 4A, 4B et 4C);
- *K. planticola* (Figs.5A, 5B )
- *S. aureus* (Figs. 6A, 6B et 6C)
- *E.faecalis* (Figs.7A, 7B, 7C).

Les pentes nulles obtenues par régression linéaire montrent que pour les quatre germes étudiés, les lots sont stables au moins:
- 1 semaine à +4°C:
- 3 mois à - 20°C;
- 1 an à - 70°C.

Ces stabilités sont tout à fait compatibles avec une utilisation industrielle.

### EXEMPLE 5:

### Utilisation des pastilles lenticulées en tant que matériau de référence.

Les matériaux de référence peuvent être utilisés pour le contrôle de fertilité des milieux de culture, la maîtrise statistique des procédés et les essais d'aptitude par comparaison entre laboratoires.

### 1. Contrôle de la fertilité des milieux de culture.

Les normes AFNOR T90-432 et T90-433 imposent des critères de qualité pour la fabrication du milieu de culture en microplaque.

Le contrôle de qualité doit porter sur chaque lot de microplaques fabriquées. Les microplaques à tester sont prélevées de manière aléatoire ou systématique afin de constituer un échantillon selon la norme AFNOR NFX 06-023, en respectant le niveau normal de prélèvement (9 microplaques contrôlées pour une taille de lot de 1000 microplaques fabriquées).

La fertilité du milieu de culture est mesurée par le rapport entre le nombre de micro-organismes observés avec le lot de microplaques testées et le nombre de micro-organismes attendus avec un matériau de référence stable (valeur-cible). Le niveau de concentration à mettre en oeuvre après reconstitution du matériau de référence doit se situer autour du maximum de précision de la méthode, soit environ 500 germes/100 ml. Les seuils d'acceptation du lot de microplaques en essai sont : 0,66 à 1,50 fois la valeur-cible (66% < rendement (%) < 150%).

La souche à tester pour la norme T90-433 est *E. coli* WR1.

Les souches à tester pour la norme T90-432 sont *E. faecium* WR63, *E. faecalis* CCM 2541 et *E. hirae* CCM 2423

Les tableaux 3 à 6 rapportent pour chacune des souches les résultats de fertilité obtenus sur la fabrication de microplaques de 1997. Ils montrent une parfaite maîtrise de la fabrication par le producteur. En effet, 90% des lots fabriqués répondent au critère de fertilité fixé dans les normes.

### 2. Maîtrise statistique des procédés (contrôle interne de la qualité).

Avant la mise en place du contrôle interne de la qualité, le laboratoire doit étalonner sa chaîne analytique. Pour cela, afin de tenir compte des résultats obtenus dans les exemples précédents, les protocoles suivants ont été arrêtés:

### Protocole de reconstitution des matériaux de référence:

- Sortir le (s) tube(s) du congélateur.
- A l'aide d'une pince stérile, prélever la (les) pastille(s) nécessaire(s) au contrôle (pastilles lenticulées fabriquées comme décrit dans l'exemple 1).
- Replacer immédiatement le(s) tube(s) au congélateur.
- Laisser la (les) pastille(s) 15 minutes à température ambiante.
- Plonger chaque pastille dans un tube contenant 18 ml de diluant spécial pastille (suspension mère).
- Laisser 15 minutes à température ambiante sans agiter.
- Agiter doucement le(s) tube(s) durant 30 secondes.
- Si les ensemencements ne sont pas réalisés dans l'heure qui suit la reconstitution, conserver le(s) tube(s) de suspension mère dans la glace fondante (garantie d'une stabilité de quatre heures).

### Protocole de préparation du diluant spécial pastilles:

- Dissoudre 22,5g de sels marins (Instant Ocean) dans 1 litre d'eau distillée,
- vérifier la salinité (20+/-3 pour 1000) ou la conductivité (25000 +/-4000 uS.cm-1),
- ajuster le pH (7.9+/-0.5).
- répartir en tubes de 18 ml.
- autoclaver à 120°C pendant 15 minutes.

### Protocole de calibration:

- Utiliser la suspension mère:
   - Les pastilles 1 et 2 : filtrer 0.5 ml,
   - Les pastilles 3 et 4: filtrer 1.0 ml,
   - Les pastilles 5 et 6: filtrer 2.0 ml,
   - Les pastilles 7 et 8: filtrer 4.0 ml,
   - Les pastilles 9 et 10: filtrer 8.0 ml.
- Vérifier la linéarité des résultats par régression linéaire. A partir de l'équation de la droite de régression, déterminer le volume de filtration à préconiser pour chaque matériau de manière à obtenir 25 colonies sur la membrane.

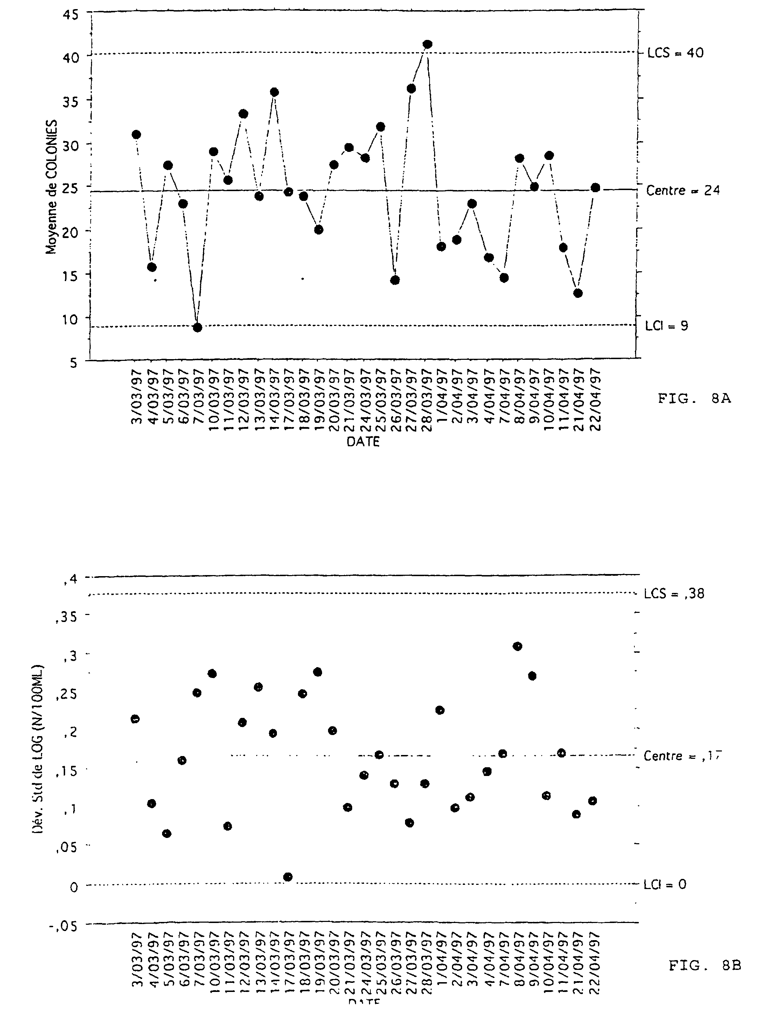

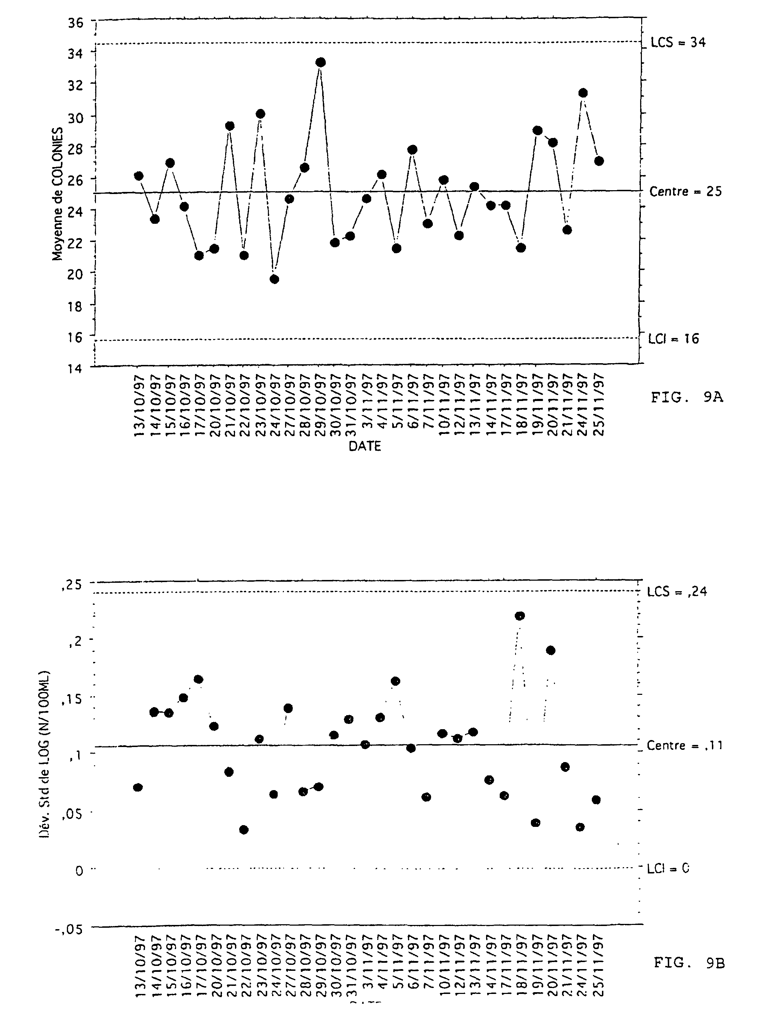

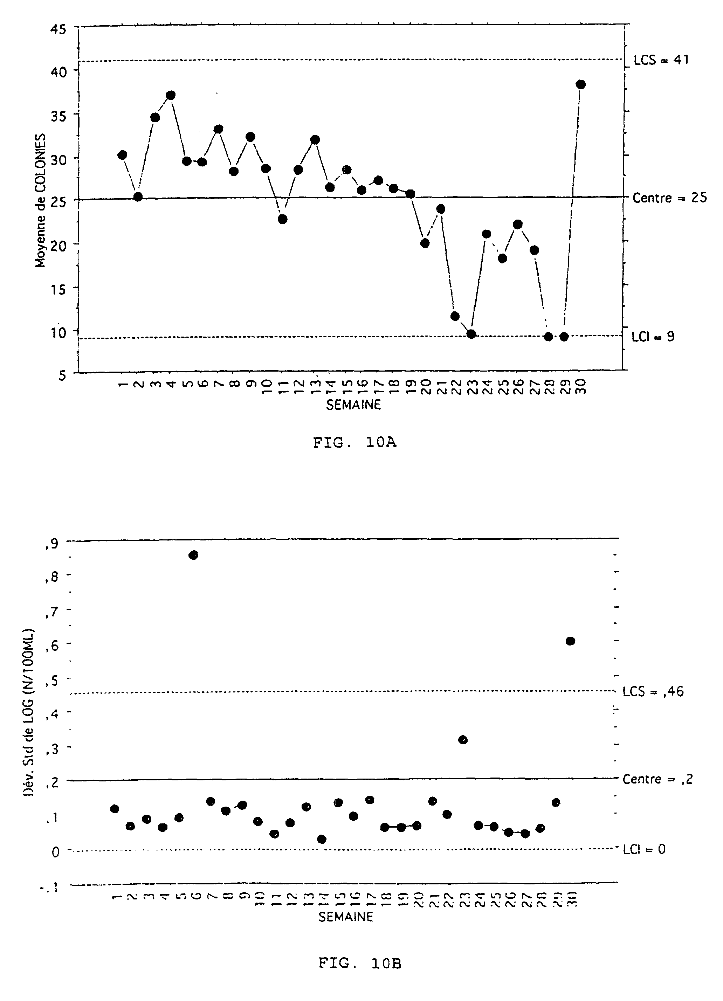

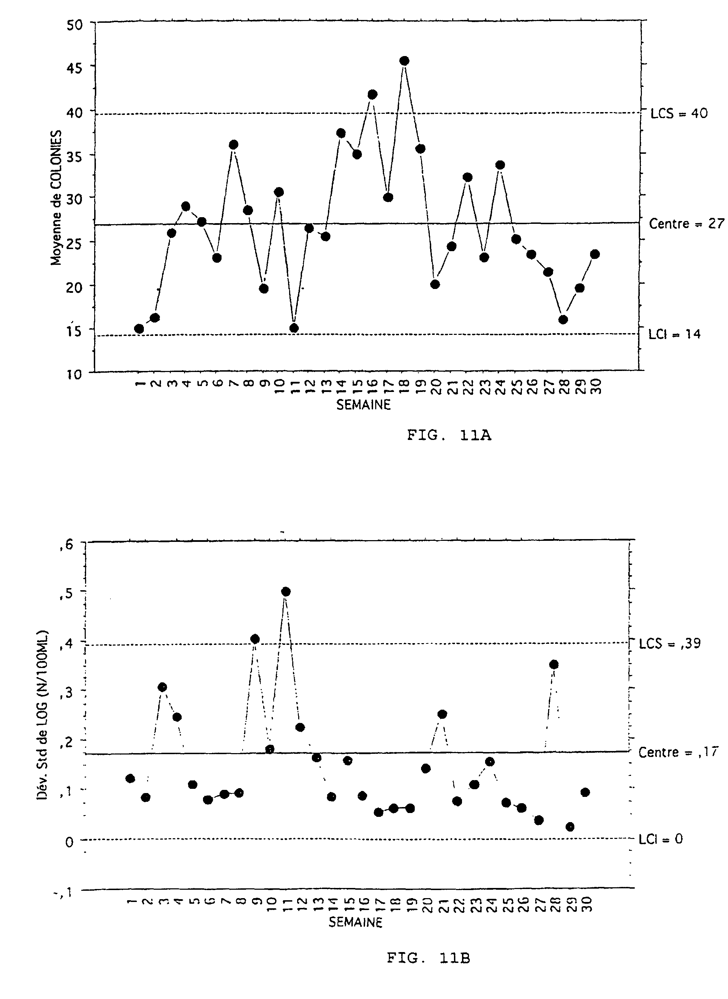

Une fois centré sur cette valeur cible de 25 colonies, le laboratoire doit mettre en place, afin de déceler des dérives, des décentrages, des problèmes de répétabilité ou de reproductibilité, son contrôle interne. Selon l'activité analytique du laboratoire, ce contrôle interne assure:
- Une couverture journalière:
   Les gros laboratoires réalisant une centaine d'analyses par jour peuvent tester 4 pastilles chaque jour. Les figures 8A et 8B; 9A et 9B représentent les cartes de contrôle de la moyenne et de l'écart type obtenues en routine avec des pastilles selon l'invention contenant *E. coli* et *E. faecalis* sur une période de 30 jours.
- Une couverture hebdomadaire:
   Les petits laboratoires réalisant une centaine d'analyses par semaine peuvent tester 5 pastilles chaque semaine à raison d'une pastille par jour. Les figures 10A et 10B et 11A et 11B, représentent les cartes de contrôle de la moyenne et de l'écart type obtenues en routine avec des pastilles selon l'invention contenant *S. aureus* et *C. sporogenes* sur une période de 30 semaines.
   Grâce à ce type de contrôle interne les laboratoires de microbiologie disposent maintenant d'un système d'alerte en temps réel (couverture quotidienne) ou en temps différé (couverture hebdomadaire) avec possibilité de sélectionner les causes d'erreurs et mettre en place les actions correctives.

### 3. Essais d'aptitude des laboratoires par intercomparaison (contrôle externe de la qualité).

Un essai d'aptitude consiste à utiliser des intercomparaisons pour déterminer la performance d'un laboratoire en matière d'essais ou de mesurages. La participation à des systèmes d'essais d'aptitude donne aux laboratoires un moyen objectif d'évaluer et de démontrer la fiabilité des données qu'ils produisent. Les laboratoires complètent ainsi les procédures internes de maîtrise de la qualité en fournissant une mesure externe supplémentaire de leurs compétences en matières d'essais.

L'Association Générale des Laboratoires d'Analyse de l'Environnement (A.G.L.A.E.) a organisé en Avril 98 un essai interlaboratoires regroupant 55 laboratoires français (métropole et départements d'Outre-mer). Des pastilles fabriquées comme décrit dans l'exemple 1 ont été utilisées comme matériau d'essai.

Les résultats obtenus lors d'un dénombrement des entérocoques sur un lot de pastilles contenant *Enterococcus faecalis* sont résumés dans le tableau 7.

Dans le tableau 7 ci-après, Sᵤ mesure la variabilité des résultats obtenus quand les mesures sont effectuées sur la même pastille au sein d'un même laboratoire.

r correspond à la variabilité des résultats obtenus quand les mesures sont effectuées sur des pastilles différentes au sein d'un même laboratoire.

R mesure la variabilité des résultats obtenus quand les mesures sont effectuées sur des pastilles différentes et par différents laboratoires.

La valeur de 0,10 obtenue pour Sᵤ montre que les pastilles se dissolvent de manière homogène.

La valeur de 0,18 obtenue pour r montre la bonne homogénéité du lot de pastilles.

La valeur de 0,3 obtenue pour R est une valeur comparable aux résultats habituellement obtenus dans ce type d'essai inter-laboratoires, et valide donc cette technique (stabilité des pastilles lors du transfert et mise en oeuvre aisée dans les laboratoires).

L'intérêt de la présente invention dans le cadre de ces essais inter-laboratoires réside dans la possibilité de conserver, même à température ambiante, les pastilles. Ainsi, les essais inter-laboratoires effectués les années précédentes nécessitaient de fournir à chaque laboratoire un échantillon consistant en un litre d'eau artificiellement contaminé. Cet échantillon devait donc parvenir en quelques heures à chaque laboratoire, afin d'être certain que tous les laboratoires travaillaient dans les mêmes conditions.

Les pastilles selon la présente invention permettent de résoudre ce problème et facilitent l'organisation de ce type d'essai.

**TABLEAU 1**

| Séchage | Nombre moyen de colonies sur 10 boîtes |
|---|---|
| + 44°C | 24 |
| sous hotte à flux laminaire | > 100 |
| - 20°C | > 100 (*) |

| | |
|---|---|
| (*) Boîtes deux fois plus chargées qu'à température ambiante. | |

**TABLEAU 2**

| | E.coli | K.planticola | E.faecalis | S.aureus | C.sporogenes |
|---|---|---|---|---|---|
| Mini= | 8 | 6 | 8 | 28 | 8 |
| Moyenne = | 24 | 22 | 18 | 61 | 19 |
| Maxi= | 70 | 83 | 38 | 135 | 44 |
| SR | 0,16 | 0,19 | 0,11 | 0,11 | 0,12 |

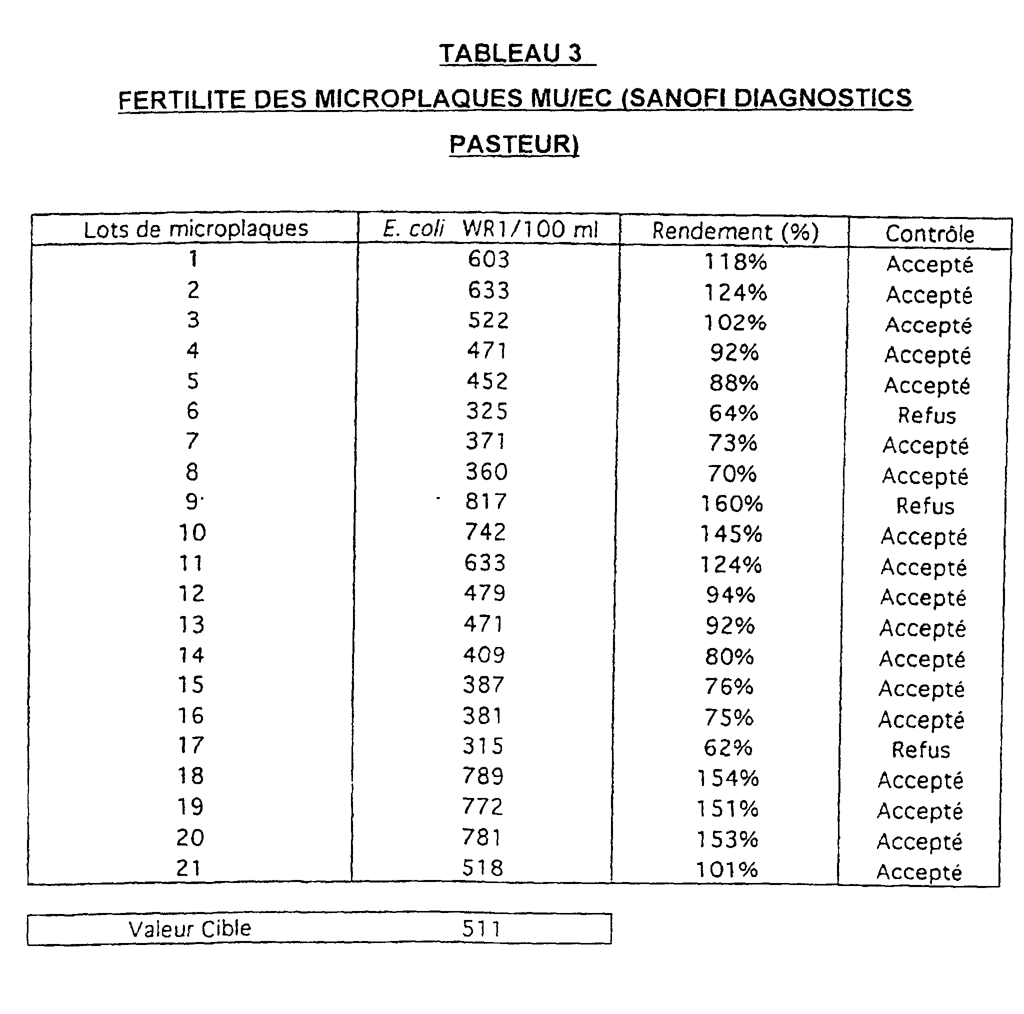

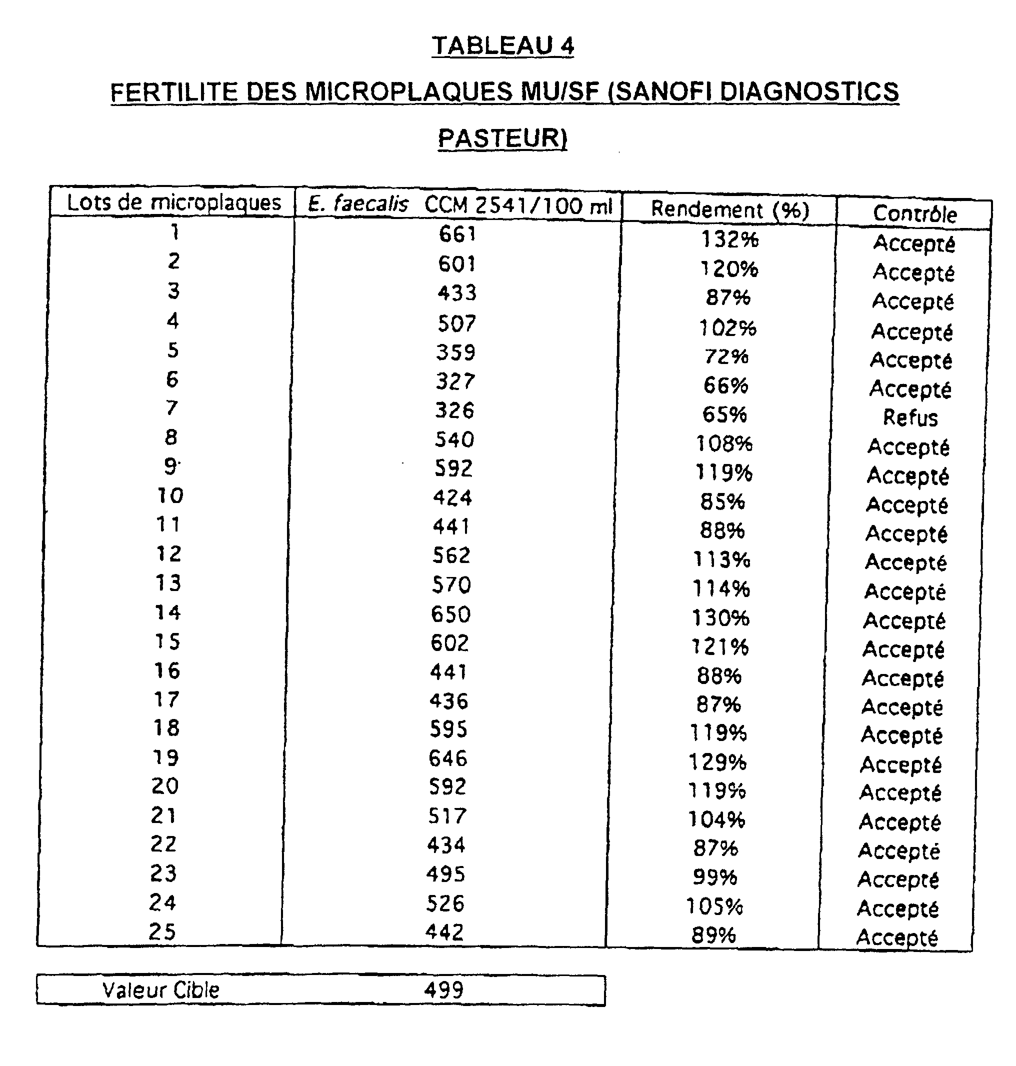

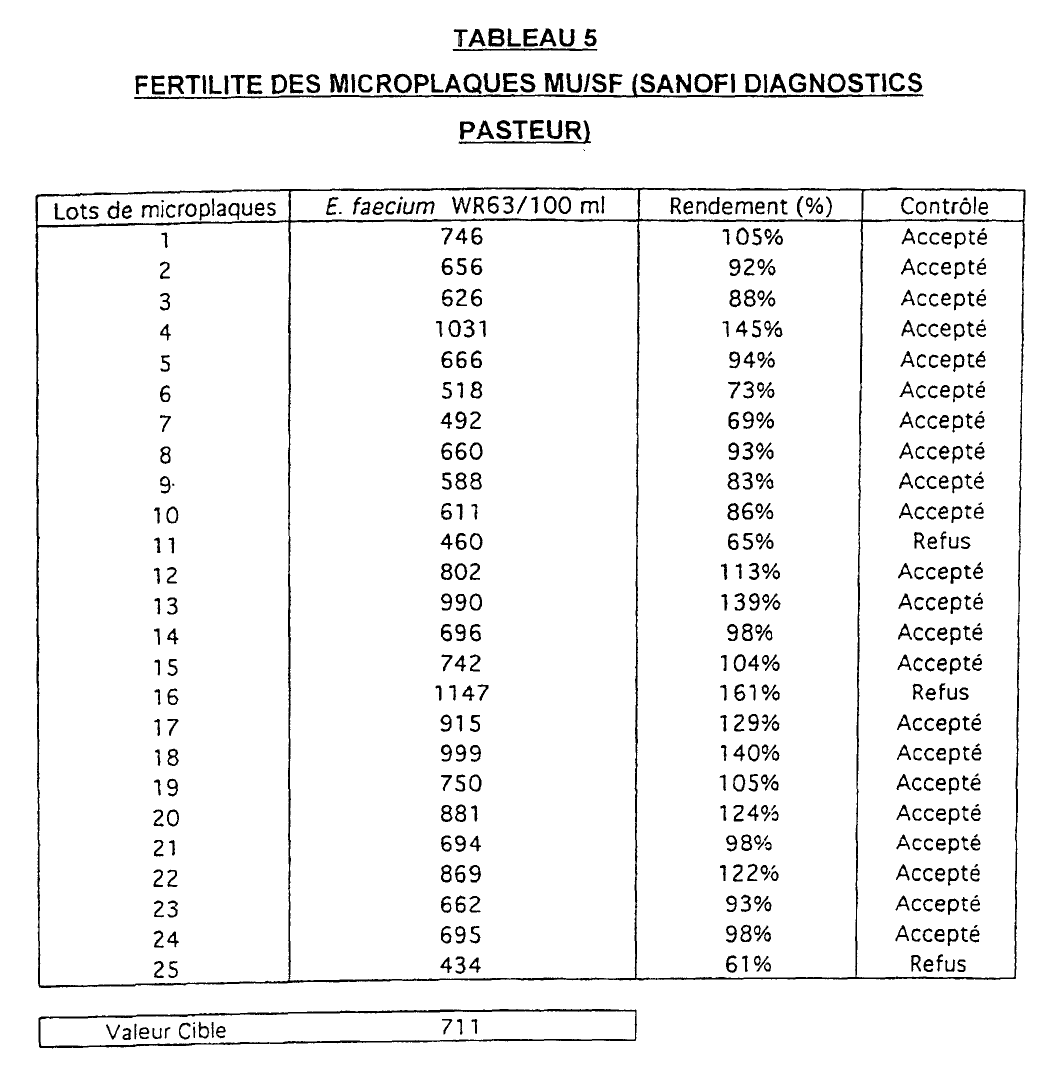

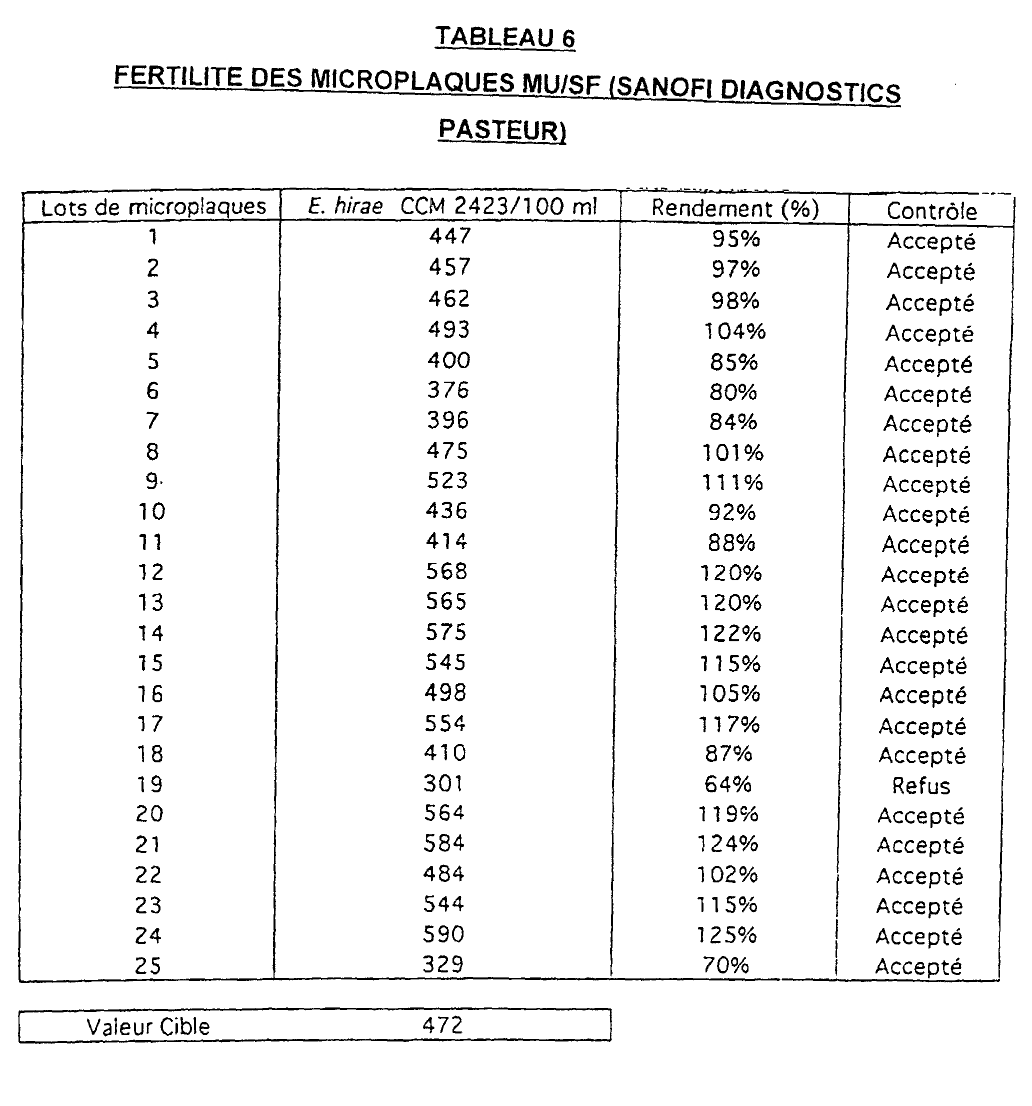

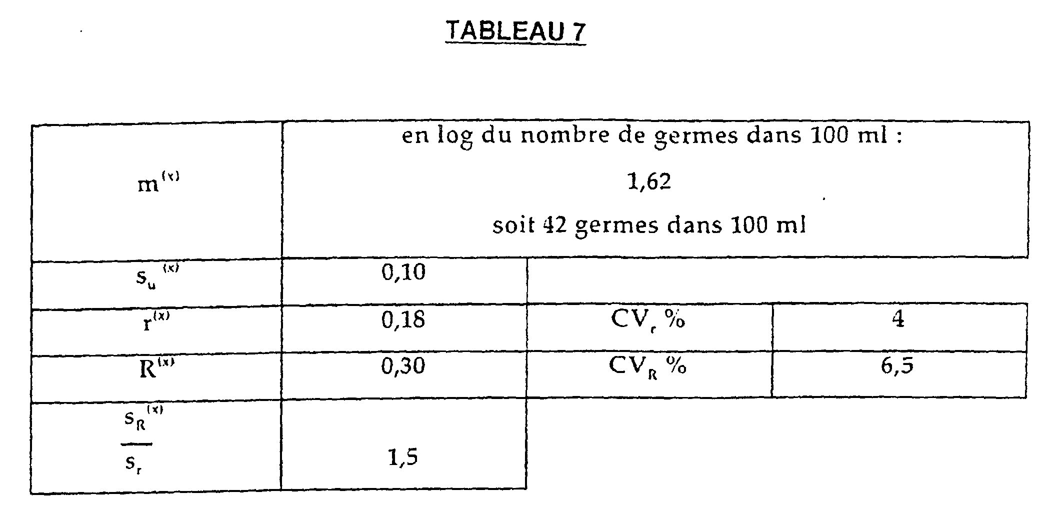

## Revendications

1. Pastilles pour la conservation de micro-organismes susceptibles d'être obtenues par séchage sous vide à une température inférieure à 10°C d'une composition comprenant:
(1) un mélange d'albumine et d'amidon contenant entre 20 et 40% d'albumine en poids total de la composition et entre 2 et 5% d'amidon en poids total de la composition,
(2) entre 60 et 80% de saccharose en poids total de la composition, et
(3) 100 à 10¹¹ micro-organismes par gramme de la composition.

2. Pastilles selon la revendication 1, **caractérisées en ce que** l'albumine est de l'ovalbumine.

3. Pastilles selon l'une quelconque des revendications 1 et 2, **caractérisées en ce que** les micro-organismes sont des bactéries, des virus, des levures, des protozoaires ou des champignons.

4. Pastilles selon l'une quelconque des revendications 1 à 3, **caractérisées en ce qu'**elles présentent une masse comprise entre 1 et 250 mg, préférentiellement entre 2 et 100 mg et encore plus préférentiellement entre 10 et 25 mg.

5. Pastilles selon la revendication 1, **caractérisées en ce qu'**elles ont un diamètre compris entre 1 et 10 mm.

6. Procédé de fabrication de pastilles selon l'une quelconque des revendications 1 à 5, comprenant les étapes suivantes:
- incorporation des micro-organismes au mélange d'albumine et d'amidon,
- diminution de l'activité en eau par ajout progressif de saccharose,
- mise en forme des pastilles, et
- séchage des pastilles sous vide et à une température inférieure à environ -10°C.

7. Procédé selon la revendication 6, **caractérisé en ce que** le séchage des pastilles est effectué dans un dessiccateur durant entre 12 heures et 10 jours.

8. Procédé de reconstitution d'une suspension de micro-organismes à partir de pastilles selon la revendication 1, ou obtenues par le procédé selon l'une quelconque des revendications 6 et 7, **caractérisé en ce que** les pastilles sont remises en suspension dans un milieu adéquat.

9. Procédé selon la revendication 8, **caractérisé en ce que** les pastilles sont reconstituées dans un milieu liquide contenant 23 g/l de sel de mer.

10. Utilisation de pastilles selon l'une quelconque des revendications 1 à 5 comme matériau de référence en microbiologie.

11. Utilisation de pastilles selon l'une quelconque des revendications 1 à 5 pour le contrôle de fertilité des milieux de culture.

12. Utilisation de pastilles selon l'une quelconque des revendications 1 à 5 en tant que témoins positifs dans les "challenge tests".

13. Utilisation de pastilles selon l'une quelconque des revendications 1 à 5 en tant qu'agents de démarrage dans les processus de fermentation.

14. Utilisation de pastilles selon l'une quelconque des revendications 1 à 5 comme matériau de référence dans le domaine de la bactériologie des eaux et l'agroalimentaire.

## Claims

1. Pellets for the conservation of microorganisms, which can be obtained by drying, under vacuum at a temperature of less than 10°C, a composition comprising:
(1) a mixture of albumin and starch containing between 20 and 40% of albumin by total weight of the composition and between 2 and 5% of starch by total weight of the composition,
(2) between 60 and 80% of sucrose by total weight of the composition, and
(3) 100 to 10¹¹ microorganisms per gram of the composition.

2. Pellets according to Claim 1, **characterized in that** the albumin is ovalbumin.

3. Pellets according to either one of Claims 1 and 2, **characterized in that** the microorganisms are bacteria, viruses, yeast, protozoa or fungi.

4. Pellets according to any one of Claims 1 to 3, **characterized in that** they have a mass of between 1 and 250 mg, preferentially between 2 and 100 mg, and even more preferentially between 10 and 25 mg.

5. Pellets according to Claim 1, **characterized in that** they have a diameter of between 1 and 10 mm.

6. Method for producing pellets according to any of Claims 1 to 5, comprising the following steps:
- incorporating the microorganisms into the mixture of albumin and starch,
- decreasing the water activity by gradually adding sucrose,
- formulating the pellets, and
- drying the pellets under vacuum at a temperature of less than approximately -10°C.

7. Method according to Claim 6, **characterized in that** the drying of the pellets is carried out in a desiccator for between 12 hours and 10 days.

8. Method for reconstituting a suspension of microorganisms from the pellets according to Claim 1, or obtained using the method according to either one of Claims 6 and 7, **characterized in that** the pellets are resuspended in a suitable medium.

9. Method according to Claim 8, **characterized in that** the pellets are reconstituted in a liquid medium containing 23 g/l of sea salt.

10. Use of pellets according to any one of Claims 1 to 5, as reference material in microbiology.

11. Use of pellets according to any one of Claims 1 to 5, for the control of fertility of culture media.

12. Use of pellets according to any one of Claims 1 to 5, as positive controls in challenge tests.

13. Use of pellets according to any one of Claims 1 to 5, as initiating agents in fermentation processes.

14. Use of pellets according to any one of Claims 1 to 5, as reference material in the field of water bacteriology and agrofoods.

## Patentansprüche

1. Pastillen für die Aufbewahrung von Mikroorganismen, die erhalten werden können durch Trocknung unter Vakuum bei einer Temperatur unter 10°C einer Zusammensetzung, umfassend:
(1) eine Mischung von Albumin und Stärke, die zwischen 20 und 40% Albumin bezogen auf das Gesamtgewicht der Zusammensetzung und zwischen 2 und 5% Stärke bezogen auf das Gesamtgewicht der Zusammensetzung enthält,
(2) zwischen 60 und 80% Saccharose bezogen auf das Gesamtgewicht der Zusammensetzung und
(3) 100 bis 10¹¹ Mikroorganismen pro Gramm der Zusammensetzung.

2. Pastillen nach Anspruch 1, **dadurch gekennzeichnet, dass** das Albumin Ovalbumin ist.

3. Pastillen nach einem der Ansprüche 1 und 2, **dadurch gekennzeichnet, dass** die Mikroorganismen Bakterien, Viren, Hefen, Protozoen oder Pilze sind.

4. Pastillen nach einem der Ansprüche 1 bis 3, **dadurch gekennzeichnet, dass** sie eine Masse zwischen 1 und 250 mg, vorzugsweise zwischen 2 und 100 mg und noch mehr bevorzugt zwischen 10 und 25 mg aufweisen.

5. Pastillen nach Anspruch 1, **dadurch gekennzeichnet, dass** sie einen Durchmesser zwischen 1 und 10 mm haben.

6. Verfahren zur Herstellung von Pastillen nach einem der Ansprüche 1 bis 5, welches die folgenden Schritte umfasst:
- Einarbeiten der Mikroorganismen in die Mischung von Albumin und Stärke,
- Verringerung der Aktivität in Wasser durch fortschreitende Zugabe von Saccharose,
- Formung der Pastillen und
- Trocknung der Pastillen unter Vakuum und bei einer Temperatur unter ungefähr -10°C.

7. Verfahren nach Anspruch 6, **dadurch gekennzeichnet, dass** die Trocknung der Pastillen in einem Exsiccator während zwischen 12 h und 10 Tagen bewirkt wird.

8. Verfahren zur Rekonstitution einer Suspension von Mikroorganismen ausgehend von Pastillen nach Anspruch 1 oder erhalten durch das Verfahren gemäß einem der Ansprüche 6 und 7, **dadurch gekennzeichnet, dass** die Pastillen in einem adäquaten Medium resuspendiert werden.

9. Verfahren nach Anspruch 8, **dadurch gekennzeichnet, dass** die Pastillen in einem flüssigen Medium, das 23 g/l Meersalz enthält, rekonstituiert werden.

10. Verwendung von Pastillen nach einem der Ansprüche 1 bis 5 als Referenzmaterial in der Mikrobiologie.

11. Verwendung von Pastillen nach einem der Ansprüche 1 bis 5 für die Fertilitätskontrolle von Kulturmedien.

12. Verwendung von Pastillen nach einem der Ansprüche 1 bis 5 als positive Vergleichsproben in "challenge tests".

13. Verwendung von Pastillen nach einem der Ansprüche 1 bis 5 als Startermittel in Fermentationsverfahren.

14. Verwendung von Pastillen nach einem der Ansprüche 1 bis 5 als Referenzmaterial auf dem Gebiet der Bakteriologie der Wässer und der Agrarwirtschaft.
